(19) Europäisches Patentamt
European Patent Office
Office européen des brevets

(11) **EP 1 488 699 B1**

(12) **EUROPEAN PATENT SPECIFICATION**

(45) Date of publication and mention
of the grant of the patent:
**16.01.2013  Bulletin 2013/03**

(51) Int Cl.:
*A01N 43/80* (2006.01)     *A01N 43/80* (2006.01)
*A01N 39/00* (2006.01)

(21) Application number: **04255358.6**

(22) Date of filing: **18.01.2003**

(54) **Synergistic microbicidal combinations**

Synergistische mikrobizide Kombinationen

Associations microbicides synergiques

(84) Designated Contracting States:
**DE FR GB IT**

(30) Priority:  **31.01.2002  US 352621 P**

(43) Date of publication of application:
**22.12.2004  Bulletin 2004/52**

(62) Document number(s) of the earlier application(s) in
accordance with Art. 76 EPC:
**03250329.4 / 1 332 675**

(73) Proprietor: **ROHM AND HAAS COMPANY**
**Philadelphia,**
**Pennsylvania 19106-2399 (US)**

(72) Inventors:
• **Warwick, Eileen Fleck**
**Lansdale**
**Pannsylvania 19446 (US)**
• **Diehl, Megan Anne**
**Line Lexington**
**Pennsylvania 18932 (US)**

(74) Representative: **Buckley, Guy Julian et al**
**Patent Outsourcing Limited**
**1 King Street**
**Bakewell**
**Derbyshire DE45 1DZ (GB)**

(56) References cited:
EP-A- 0 450 916     EP-A- 0 745 324
EP-A- 0 773 281     EP-A- 1 082 901
WO-A-01/00022      GB-A- 2 138 799

**Description**

This invention relates to synergistic microbicidal combinations and in particular, to synergistic microbicidal combinations of 2-methyl-3-isothiazolone with phenoxy ethanol , where the resulting composition is substantially free of halogenated 3-isothiazolone.

Kathon™ CG biocide (3/1 ratio of 5-chloro-2-methyl-3-isothiazolone to 2-methyl-3-isothiazolone) and Neolone™ 950 bactericide (2-methyl-3-isothiazolone) are highly effective preservatives and may be used in combination with a variety of commercially available preservatives to prevent microbial contamination in personal care applications, such as cosmetics and toiletries (see "Kathon™ CG Cosmetic and Toiletry Preservative" Bulletin CS-663, September 1997, and " Neolone™ 950 Preservative for Personal Care" Bulletin CS-707, May 2001, Rohm and Haas Company, Philadelphia, PA). U.S. Patent No. 5,591,759 discloses the use of 1,2-dibromo-2,4-dicyanobutane to stabilize isothiazolone mixtures of 5-chloro-2-methyl-3-isothiazolone/2-methyl-3-isothiazolone (9/1 ratio). Patent application WO 01/50855 discloses preservative formulations for extending the lifetime of cut flowers based on preservative formulations containing acrylic copolymer additives, saccharide derivatives and antimicrobial mixtures, including those based on 2-methyl-3-isothiazolone in the presence of citric acid/citrates. EP-A-1082901 discloses stable liquid microbicide compositions comprising 60 to 95% of at least one water-soluble, non-halogenated 3-isothiazolone and 5 to 40% of at least one solvent selected from water, (C$_1$ - C$_4$) alkanol, water-miscible (C$_2$ - C$_8$) alkoxy/hydroxy compound and water-miscible (C$_2$ - C$_4$) aprotic compound. EP-A-0450916 discloses the use of phenoxyalkanol compounds as a stabilizer for isothiazolone compositions. EP-A-0745324 discloses stabilized 3-isothiazolone compositions comprising organic solvents having less than 6 percent by weight solubility in water. WO 01/00022 A1 discloses an anti-contamination composition comprising 2-methyl-3-isothiazolone and phenoxyethanol, wherein the ratio of 2-methyl-3-isothiazolone to phenoxyethanol is 1:2. GB-A-2138799 discloses a method for the treatment of an aqueous system comprising adding an ethoxylated phenol and a biocide to water. EP-A-0773281 discloses an additive mixture containing an N-(cyclo)alkyl-isothiazolone, a stabilizer and a solubiliser selected from ohenoxyethanol, phenoxypropanols, phenoxybutanols, dipropylene glycol, 1-methoxypropan-2-ol and butyl diglycol.

Many other microbicidal agents are known. These are commercially available for the control of microorganisms in a variety of loci. Sometimes, many microbicides cannot provide effective antimicrobial control even at high use concentrations due to weak activity against certain microorganisms. Without effective microorganism control, loss of product, inferior product, production time loss, health hazard and other problems may occur in the locus treated. There is a need for a method to control various microorganisms that does not rely on high use levels of combinations of different microbicidal agents, but still provides effective overall control of the microorganisms that is both quick and long lasting.

The problem addressed by the present invention is to overcome deficiencies of previous microbicide combinations by providing a combination of microbicidal agents that is more effective than the individual microbicides used alone and that can be used at lower overall levels while providing efficacy similar to the original individual microbicide levels.

STATEMENT OF INVENTION

The present invention, in its various aspects, is as set out in the accompanying claims.

The present invention provides a microbicidal composition comprising a synergistic mixture, the first component of which is 2-methyl-3-isothiazolone, and the second component of which is phenoxyethanol; wherein the ratio of the first component to the second component is from 1/30 to 1/80 or 1/107 to 1/133; and wherein the composition is substantially free of halogenated 3-isothiazolone.

The present invention further provides a non-therapeutic method of inhibiting the growth of *P. aeruginosa* in a locus comprising introducing to, at or on, the locus a microorganism inhibiting amount of the aforementioned synergistic mixture; and wherein the amount of synergistic mixture is from 0.1 to 10,000 parts per million active ingredient.

DETAILED DESCRIPTION

We have discovered that 2-methyl-3-isothiazolone (MI) may be combined with phenoxyethanol to provide enhanced microbicidal efficacy at a combined active ingredient level lower than that of the combined individual 3-isothiazolone or phenoxyethanol effective use levels, where the resulting composition is substantially free of halogenated 3-isothiazolone. Preferably the microbicidal compositions are also substantially free of metal salt stabilizers, such as nitrate or magnesium salts; these "salt-free" microbicidal compositions are especially useful to protect personal care compositions against microbial contamination.

It has been discovered that mixtures of MI with phenoxyethanol in a ratio of 1/30 to 1/80 or 1/107 to 1/133 result in synergistic microbicidal activities. Synergy occurs when the disruptive interaction on the organisms treated by the two compounds together is greater than the sum of such interactions of both compounds when used alone. Such synergy does not arise from the expected activity of the components when added together.

As used herein, the following terms have the designated definitions, unless the context clearly indicates otherwise. The term "microbicide" refers to a compound capable of inhibiting the growth of or controlling the growth of microorganisms at a locus; microbicides include bactericides, fungicides and algaecides. The term "microorganism" includes, for example, fungi (such as yeast and mold), bacteria and algae. The term "locus" refers to an industrial system or product subject to contamination by microorganisms. The following abbreviations are used throughout the specification: ppm = parts per million by weight (weight/weight), mL = milliliter, ATCC = American Type Culture Collection, and MIC = minimum inhibitory concentration. Unless otherwise specified, ranges listed are to be read as inclusive and combinable, temperatures are in degrees centigrade (°C), and references to percentages (%) are by weight. "Salt-free" means that the composition contains zero or up to 0.5%, preferably zero or up to 0.1%, and more preferably zero or up to 0.01%, of metal salt, based on weight of the composition.

The microbicidal compositions of the present invention are substantially free of halogenated 3-isothiazolone; that is, zero or up to 3%, preferably zero or up to 1% and more preferably zero or up to 0.5%, of halogenated 3-isothiazolone may be present, based on combined weight of halogenated 3-isothiazolone and 2-methyl-3-isothiazolone. Microbicidal compositions dependent on the presence of halogenated 3-isothiazolone are susceptible to chemical degradation and may require additional stabilizer components, such as the aforementioned metal salt stabilizers; salt stabilizers sometimes create unacceptable properties in finished formulations. For this reason it is desirable to provide microbicide formulations substantially free of halogenated 3-isothiazolone, but that still provide the degree of antimicrobial protection provided by the halogenated 3-isothiazolones; such are the microbicidal compositions of the present invention that are based on 2-methyl-3-isothiazolone, which do not require metal stabilizers.

**MI** may be used in the synergistic mixtures of the present invention "as is" or may first be formulated with a solvent or a solid carrier. Suitable solvents include, for example, water; glycols, such as ethylene glycol, propylene glycol, diethylene glycol, dipropylene glycol, polyethylene glycol, and polypropylene glycol; glycol ethers; alcohols, such as methanol, ethanol, propanol, phenethyl alcohol and phenoxypropanol; ketones, such as acetone and methyl ethyl ketone; esters, such as ethyl acetate, butyl acetate, triacetyl citrate, and glycerol triacetate; carbonates, such as propylene carbonate and dimethyl carbonate; and mixtures thereof. It is preferred that the solvent is selected from water, glycols, glycol ethers, esters and mixtures thereof. Suitable solid carriers include, for example, cyclodextrin, silicas, diatomaceous earth, waxes, cellulosic materials and charcoal. It is preferred that **MI** is formulated in water. Phenoxyethanol may be used in the synergistic mixtures of the present invention "as is" or may first be formulated together with a suitable solvent, a solid carrier or as a dispersion. Suitable solvents and solid carriers are those described above for **MI**.

When a water-insoluble second microbicide component is formulated in a solvent, the formulation may optionally contain surfactants. When such formulations contain surfactants, they are generally in the form of emulsive concentrates, emulsions, microemulsive concentrates, or microemulsions. Emulsive concentrates form emulsions upon the addition of a sufficient amount of water. Microemulsive concentrates form microemulsions upon the addition of a sufficient amount of water. Such emulsive and microemulsive concentrates are generally well known in the art; it is preferred that such formulations are free of surfactants. U.S. Patent No. 5,444,078 may be consulted for further general and specific details on the preparation of various microemulsions and microemulsive concentrates.

Any formulation of **MI** may be used with any formulation of the second microbicide component in the synergistic mixtures of the present invention. When both the **MI** and the second microbicide component are each first formulated with a solvent, the solvent used for **MI** may be the same as or different from the solvent used to formulate the other commercial microbicide. It is preferred that the two solvents are miscible. In the alternative, the **MI** and the other microbicide may be combined directly and then a solvent added to the mixture.

Those skilled in the art will recognize that the **MI** and the second microbicide component of the present invention may be added to a locus sequentially, simultaneously, or may be combined before being added to the locus. It is preferred that the **MI** and the second microbicide component be added to a locus simultaneously or combined prior to being added to the locus. When the microbicides are combined prior to being added to a locus, such combination may optionally contain adjuvants, such as, for example, solvent, thickeners, anti-freeze agents, colorants, sequestrants (such as ethylenediaminetetraacetic acid, ethylenediaminedisuccinic acid, iminodisuccinic acid and salts thereof), dispersants, surfactants, stabilizers, scale inhibitors and anti-corrosion additives.

The microbicidal compositions of the present invention can be used to inhibit the growth of microorganisms by introducing a microbicidally effective amount of the compositions onto, into, or at a locus subject to microbial attack. Suitable loci include, for example: cooling towers; air washers; boilers; mineral slurries; wastewater treatment; ornamental fountains; reverse osmosis filtration; ultrafiltration; ballast water; evaporative condensers; heat exchangers; pulp and paper processing fluids; plastics; emulsions; dispersions; paints; latexes; coatings, such as varnishes; construction products, such as mastics, caulks, and sealants; construction adhesives, such as ceramic adhesives, carpet backing adhesives, and laminating adhesives; industrial or consumer adhesives; photographic chemicals; printing fluids; household products, such as bathroom and kitchen cleaners; cosmetics; toiletries; shampoos; soaps; detergents; industrial cleaners; floor polishes; laundry rinse water; metalworking fluids; conveyor lubricants; hydraulic fluids; leather and leather products; textiles; textile products; wood and wood products, such as plywood, chipboard, flakeboard, laminated beams, oriented strandboard, hardboard, and particleboard; petroleum processing fluids; fuel; oilfield fluids, such as injection water, fracture fluids, and drilling muds; agriculture adjuvant preservation; surfactant preservation; medical devices; diagnostic reagent preservation; food preservation, such as plastic or paper food wrap; pools; and spas.

Preferably, the microbicidal compositions of the present invention are used to inhibit the growth of microorganisms at a locus selected from one or more of emulsions, dispersions, paints, latexes, household products, cosmetics, toiletries, shampoos, soaps, detergents and industrial cleaners. In particular, the microbicidal compositions are useful in personal care applications, such as hair care (for example, shampoo and dyes) and skin care (for example, sunscreens, cosmetics, soaps, lotions and creams) formulations.

When the synergistic compositions of the present invention are used in personal care compositions, the formulated compositions may also comprise one or more ingredients selected from UV radiation-absorbing agents, surfactants, rheology modifiers or thickeners, fragrances, moisturizers, humectants, emollients, conditioning agents, emulsifiers, antistatic aids, pigments, dyes, tints, colorants, antioxidants, reducing agents and oxidizing agents.

Suitable UV radiation-absorbing agents (including chemical absorbers and physical blockers) include, for example, oxybenzone, dioxybenzone, sulisobenzone, menthyl anthranilate, para-aminobenzoic acid, amyl para-dimethylaminobenzoic acid, octyl para-dimethylaminobenzoate, ethyl 4-bis(hydroxypropyl)aminobenzoate, polyethylene glycol (PEG-25) para-aminobenzoate, diethanolamine para-methyoxycinnamate, 2-ethoxyethyl para-methoxycinnamate, ethylhexyl para-methoxycinnamate, octyl para-methoxycinnamate, isoamyl para-methoxycinnamate, 2-ethylhexyl-2-cyano-3,3-diphenylacrylate, 2-ethylhexyl salicylate, homomenthyl salicylate, glyceryl aminobenzoate, triethanolamine salicylate, digalloyl trioleate, lawsone with dihydroxyacetone, 2-phenylbenzimidazole-5-sulfonic acid, 4-methylbenzylidine camphor, avobenzone, titanium dioxide and zinc oxide. Alternatively, UV radiation-absorbing agents such as triazines, benzotriazoles, vinyl group-containing amides, cinnamic acid amides and sulfonated benzimidazoles may also be used.

Suitable surfactants include, for example, nonionic, anionic, cationic and amphoteric surfactants and mixtures thereof; such as PPG 28 Buteth 35, PEG 75 lanolin, perfluoropolymethyl isopropyl ether, octoxynol-9, PEG-25 hydrogenated castor oil, polyethylene terephthalate, polyethylene glycol 25 glyceryl trioleate, oleth-3 phosphate, PPG-5-ceteth-10 phosphate, PEG-20 methyl glucose ether, glycereth-7-triacetate and N-alkyl substituted lactam (such as N-octyl pyrrolidone).

Suitable thickeners or rheology modifiers include, for example, hydrophobically modified nonionic ethoxylated urethanes, polycarboxylic acid thickeners such as acrylates/steareth-20 methacrylate copolymer, carbomers, acrylates copolymer and acrylates $C_{10-30}$ alkyl acrylate crosspolymer.

The personal care compositions improved by the method of this invention include, for example:

(a) hair care formulations, including shampoos, hair dyes, hair conditioners, gels, mousses and hair sprays; and
(b) skin care and nail care formulations, including nail coatings, cosmetics, astringents, depilatories, facial make-up formulations, sunscreens and sunblocks, premoistened wipes, hand creams, hand and body soaps, and hand and body lotions.

The cosmetic formulations typically contain water, film forming materials, emulsifiers, softeners, emollients, oils, stabilizers, thickeners, neutralizers, perfume, colorants, pigments and combinations thereof. The sunscreen formulations typically contain UV radiation-absorbing agents, water, film forming materials, emulsifiers, emollients, waterproofing agents, oils, stabilizers, thickeners, preservatives, perfume, colorants, insecticides, humectants and combinations thereof.

Optionally, other additives, such as additional film forming agents, plasticizers, antifoaming agents, leveling aids, excipients, vitamins, natural extracts, proteins, sequestrants, dispersants, antioxidants, suspending agents and solvents may be added to the personal care formulations described above. Suitable solvents include, for example, $C_1$-$C_{12}$ straight or branched chain alcohols such as ethanol, isopropanol, or propanol; alkyl esters such as ethyl acetate; ketones; and combinations thereof.

The specific amount of the synergistic combinations necessary to inhibit or control the growth of microorganisms in a locus depends upon the particular compounds in the combination and particular locus to be protected. Typically, the amount of the synergistic combinations of the present invention to control the growth of microorganisms in a locus is sufficient if it provides from 0.1 to 10,000 ppm active ingredient of the synergistic mixture in the locus. It is preferred that the synergistic mixture be present in an amount of 0.5 to 5000 ppm, and more preferably from 1 to 3000 ppm, in the locus.

Microorganisms evaluated for effectiveness of control by the compositions of the present invention include bacterial (*P. aeruginosa* and *S. aureus)* and fungal (*A. niger* and *C. albicans)* microorganisms, in particular Gram (-) bacteria and Gram (+) bacteria, as well as yeast and mold.

Combinations of MI with phenoxyethanol were particulary effective against both Gram (-) and Gram (+) bacteria. Combinations of MI with phenoxyethanol were particulary effective against both yeast and mold. Combinations of MI with phenoxyethanol were effective against both bacterial and fungal microorganisms.

Some embodiments of the invention are described in detail in the following Examples. All ratios, parts and percentages are expressed by weight unless otherwise specified, and all reagents used are of good commercial quality unless otherwise specified. Abbreviations used in the Examples and Tables are listed below with the corresponding descriptions:

**MI** = 2-methyl-3-isothiazolone
**SI** = synergy index
**MIC** = minimum inhibitory concentration

EXAMPLES

The synergism of the combinations of the present invention was demonstrated by testing a wide range of concentrations and ratios of the compounds.

Synergism was determined by an industrially accepted method described by Kull, F.C.; Eisman, P.C.; Sylwestrowicz, H.D. and Mayer, R.L., in Applied Microbiology 9:538-541 (1961), using the ratio determined by the formula:

$$Q_a/Q_A + Q_b/Q_B = \text{Synergy Index ("SI")}$$

wherein:

$Q_A$ = concentration of compound A (first component) in ppm, acting alone, which produced an end point (MIC of Compound A).
$Q_a$ = concentration of compound A in ppm, in the mixture, which produced an end point.
$Q_B$ = concentration of compound B (second component) in ppm, acting alone, which produced an end point (MIC of Compound B).
$Q_b$ = concentration of compound B in ppm, in the mixture, which produced an end point.

When the sum of $Q_a/Q_A$ and $Q_b/Q_B$ is greater than one, antagonism is indicated. When the sum is equal to one, additivity is indicated, and when less than one, synergism is demonstrated. The lower the **SI**, the greater the synergy shown by that particular mixture. The minimum inhibitory concentration (MIC) of a microbicide is the lowest concentration tested under a specific set of conditions that prevents the growth of added microorganisms.

Synergy tests were conducted using standard microtiter plate assays with media designed for optimal growth of the test microorganism. Minimal salt medium supplemented with 0.2% glucose and 0.1% yeast extract (M9GY medium) was used for testing bacteria; Potato Dextrose Broth (PDB medium) was used for testing yeast and mold. In this method, a wide range of combinations of microbicides was tested by conducting high resolution MIC assays in the presence of various concentrations of **MI**. High resolution MICs were determined by adding varying amounts of microbicide to one column of a microtitre plate and doing subsequent tenfold dilutions using an automated liquid handling system to obtain a series of endpoints ranging from 2 ppm to 10,000 ppm active ingredient. The synergy of the combinations of the present invention was determined against two bacteria, *Staphylococcus aureus* (*S. aureus* -- ATCC #6538) or *Pseudomonas aeruginosa (P. aeruginosa* -- ATCC #9027), a yeast, *Candida albicans* (*C. albicans* -- ATCC 10231), and a mold, *Aspergillus niger* (*A. niger* -- ATCC 16404). The bacteria were used at a concentration of about 5 x 10^6 bacteria per mL and the yeast and mold at 5 x 10^5 fungi per mL. These microorganisms are representative of natural contaminants in many consumer and industrial applications. The plates were visually evaluated for microbial growth (turbidity) to determine the MIC after various incubation times at 25°C (yeast and mold) or 30 °C (bacteria).

The test results for demonstration of synergy of the microbicide combinations of the present invention are shown below in Table 1. In each test, First Component (A) was MI and the Second Component (B) was phenoxyethanol. The table shows the specific combinations of MI and the second component; results against the microorganisms tested with incubation times; the end-point activity in ppm measured by the MIC for **MI** alone ($Q_A$), for the second component alone ($Q_B$), for **MI** in the mixture ($Q_a$) and for second component in the mixture ($Q_b$); the calculated **SI** value; and the range of synergistic ratios for each combination tested (**MI**/second component or A/B)

**Table 1**

First Component = 2-methyl-3-isothiazolone

Second Component = phenoxyethanol

| Microorganism | $Q_a$ | $Q_b$ | SI | A/B |
|---|---|---|---|---|
| *A. niger* | 0 | 4000 | 1.00 | ----- |
| (72 hours) | 50 | 3000 | 0.92 | 1/60 |
| | 50 | 2000 | 0.67 | 1/40 |
| | 75 | 3000 | 1.00 | 1/40 |
| | 75 | 2000 | 0.75 | 1/27 |
| | 100 | 3000 | 1.08 | 1/30 |
| | 100 | 2000 | 0.83 | 1/20 |
| | 125 | 3000 | 1.17 | 1/24 |
| | 125 | 2000 | 0.92 | 1/16 |
| | 150 | 2000 | 1.00 | 1/13 |
| | 300 | 0 | 1.00 | ----- |
| *C. albicans* | 0 | 4000 | 1.00 | ----- |
| (72 hours) | 50 | 3000 | 1.00 | 1/60 |
| | 75 | 3000 | 1.13 | 1/40 |
| | 75 | 2000 | 0.88 | 1/27 |
| | 100 | 2000 | 1.00 | 1/20 |
| | 125 | 2000 | 1.13 | 1/16 |
| | 125 | 1000 | 0.88 | 1/8 |
| | 125 | 800 | 0.83 | 1/6.4 |
| | 125 | 600 | 0.78 | 1/4.8 |
| | 125 | 500 | 0.75 | ¼ |
| | 150 | 1000 | 1.00 | 1/6.7 |
| | 150 | 800 | 0.95 | 1/5.3 |
| | 150 | 600 | 0.90 | 1/4 |
| | 150 | 500 | 0.88 | 1/3.3 |
| | 150 | 300 | 0.83 | 1/2 |
| | 200 | 0 | 1.00 | ----- |
| *S. aureus* | 0 | 4000 | 1.00 | ----- |
| (72 hours) | 50 | 2000 | 1.13 | 1/40 |
| | 60 | 1000 | 1.00 | 1/17 |
| | 60 | 800 | 0.95 | 1/13 |
| | 80 | 0 | 1.00 | ----- |
| *P. aeruginosa* | 0 | 3000 | 1.00 | ----- |
| (24 hours) | 2.5 | 2000 | 0.83 | 1/800 |
| | 5 | 2000 | 1.00 | 1/400 |
| | 7.5 | 2000 | 1.17 | 1/267 |
| | 7.5 | 1000 | 0.83 | 1/133 |
| | 7.5 | 800 | 0.77 | 1/107 |
| | 7.5 | 600 | 0.7 | 1/80 |
| | 10 | 1000 | 1.00 | 1/100 |
| | 10 | 800 | 0.93 | 1/80 |
| | 10 | 600 | 0.87 | 1/60 |
| | 10 | 400 | 0.80 | 1/40 |
| | 10 | 300 | 0.77 | 1/30 |
| | 15 | 0 | 1.00 | ----- |

The synergistic ratios of MI/phenoxyethanol range from 1/2 to 1/800. The MI/phenoxyethanol combination showed enhanced control of Gram (+) and Gram (-) bacteria as well as yeast and mold.

**Claims**

1. A microbicidal composition comprising a synergistic mixture, the first component of which is 2-methyl-3-isothiazolone, and the second component of which is the phenoxyethanol; wherein the weight ratio of the first component to the second component is from 1/30 to 1/80 or 1/107 to 1/133; and wherein the composition has 0 or up to 3% halogenated 3-isothiazolone based on the combined weight of halogenated 3-isothiazolone and 2-methyl-3-isothiazolone.

2. The composition of claim 1, wherein the weight ratio of 2-methyl-3-isothiazolone to phenoxyethanol is from 1/30 to 1/80.

3. The composition of claim 1, wherein the weight ratio of 2-methyl-3-isothiazolone to phenoxyethanol is from 1/107 to 1/133.

4. A non-therapeutic method of inhibiting the growth of *P. aeruginosa* in a locus comprising introducing to, at or on, the locus a *P. aeruginosa* inhibiting amount of a composition as claimed in claim 1, 2 or claim 3; and wherein the amount of synergistic mixture is from 0.1 to 10,000 parts per million active ingredient.

5. The method of claim 4 wherein the amount of synergistic mixture is from 1 to 3,000 parts per million active ingredient.

**Patentansprüche**

1. Eine mikrobizide Zusammensetzung, beinhaltend eine synergistische Mischung, wobei deren erster Bestandteil 2-Methyl-3-isothiazolon ist und deren zweiter Bestandteil das Phenoxyethanol ist; wobei das Gewichtsverhältnis des ersten Bestandteils zu dem zweiten Bestandteil zwischen 1:30 und 1:80 oder 1:107 und 1:133 liegt; und wobei die Zusammensetzung 0 oder bis zu 3 % halogeniertes 3-Isothiazolon, basierend auf dem kombinierten Gewicht von halogeniertem 3-Isothiazolon und 2-Methyl-3-isothiazolon, aufweist.

2. Zusammensetzung gemäß Anspruch 1, wobei das Gewichtsverhältnis von 2-Methyl-3-isothiazolon zu Phenoxyethanol zwischen 1:30 und 1:80 liegt.

3. Zusammensetzung gemäß Anspruch 1, wobei das Gewichtsverhältnis von 2-Methyl-3-isothiazolon zu Phenoxyethanol zwischen 1:107 und 1:133 liegt.

4. Ein nicht therapeutisches Verfahren zum Inhibieren des Wachstums von *P. aeruginosa* an einem Ort, beinhaltend das Zuführen zu dem, an den oder auf den Ort einer *P. aeruginosa* inhibierenden Menge einer Zusammensetzung gemäß Anspruch 1, 2 oder Anspruch 3; und wobei die Menge an synergistischer Mischung zwischen 0,1 und 10000 Teile pro Million Wirkstoff beträgt.

5. Verfahren gemäß Anspruch 4, wobei die Menge an synergistischer Mischung zwischen 1 und 3000 Teile pro Million Wirkstoff beträgt.

**Revendications**

1. Une composition microbicide comprenant un mélange synergique, dont le premier composant est le 2-méthyl-3-isothiazolone, et dont le deuxième composant est le phénoxyéthanol ; dans laquelle le rapport en poids du premier composant sur le deuxième composant va de 1/30 à 1/80 ou de 1/107 à 1/133 ; et dans laquelle la composition a 0, ou jusqu'à 3 %, de 3-isothiazolone halogéné sur la base du poids combiné de 3-isothiazolone halogéné et de 2-méthyl-3-isothiazolone.

2. La composition de la revendication 1, dans laquelle le rapport en poids du 2-méthyl-3-isothiazolone sur le phénoxyéthanol va de 1/30 à 1/80.

3. La composition de la revendication 1, dans laquelle le rapport en poids du 2-méthyl-3-isothiazolone sur le phénoxyéthanol va de 1/107 à 1/133.

4. Un procédé non thérapeutique pour inhiber la croissance de *P. aeruginosa* dans un locus comprenant introduire dans, à ou sur le locus une quantité inhibitrice de *P. aeruginosa* d'une composition telle que revendiquée dans la revendication 1, la revendication 2 ou la revendication 3 ; et dans lequel la quantité de mélange synergique va de 0,1 à 10 000 parties par million de principe actif.

**5.** Le procédé de la revendication 4 dans lequel la quantité de mélange synergique va de 1 à 3 000 parties par million de principe actif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5591759 A
- WO 0150855 A
- EP 1082901 A
- EP 0450916 A
- EP 0745324 A
- WO 0100022 A1
- GB 2138799 A
- EP 0773281 A
- US 5444078 A

**Non-patent literature cited in the description**

- Kathon™ CG Cosmetic and Toiletry Preservative. *Bulletin CS-663,* September 1997
- Neolone™ 950 Preservative for Personal Care. Bulletin CS-707. Rohm and Haas Company, May 2001
- **KULL, F.C. ; EISMAN, P.C. ; SYLWESTROWICZ, H.D. ; MAYER, R.L.** *Applied Microbiology,* 1961, vol. 9, 538-541